# EUROPEAN PATENT APPLICATION

(11) **EP 1 250 922 A1**
(43) Date of publication of application: **23.10.2002**
(21) Application number: 00951935.6
(22) Date of filing: 10.08.2000
(51) Int. Cl.: A61K 9/70, A61K 45/00, A61P 29/00, B65H 35/07

(54) **ROLLED EXTERNAL-USE PATCH**

(30) Priority: 10.08.1999 JP 22665799; 27.01.2000 JP 2000019050
(71) Applicant: HISAMITSU PHARMACEUTICAL CO., INC., Tosu-shi Saga 841 (JP)
(72) Inventor: HIRASHIMA, Nobuchika, 408, Tashirodaikan-machi, Tosu-shi, Saga (JP); TAKATORI, Masahiro, 408, Tashirodaikan-machi, Tosu-shi, Saga (JP); SHOHO, Koki, 408, Tashirodaikan-machi, Tosu-shi, Saga (JP)
(74) Representative: Grünecker, Kinkeldey, Stockmair & Schwanhäusser Anwaltssozietät
(86) International application number: JP0005367
(87) International publication number: WO01012165

(57) **Abstract**

The external patch roll of the invention comprises a dispenser core and an external patch wrapped around the perimeter side thereof, and is provided with a pair of side covers attached to both edges of the dispenser core and covering both edge sides of the external patch roll, wherein the dispenser core and side covers are formed of plastic materials. The side covers inhibit escape of drug components from both edge sides of the roll, prevent inconveniences such as shape deformation of the roll, and enable smooth drawing out and winding up operations for the patch. Since the dispenser core and side covers are formed of plastic materials, loss of drug components due to adsorption or permeation into the materials can be greatly reduced.

## Description

### Technical Field

The present invention relates to an external patch roll comprising an external patch wrapped around a dispenser core.

### Background Art

A great variety of external patches are widely used for the purpose of ameliorating shoulder stiffness, lower back pain, muscle soreness, muscular fatigue, arthralgia and the like. Such external patches are usually constructed with a support made of a nonwoven fabric or the like, an adhesive layer laminated on one side thereof and a covering attached to the adhesive layer in a releasable manner, and include types shaped into specific sizes and roll types.

External patch rolls are composed of a dispenser core and a strip-like patch wrapped around the outer perimeter side, and their advantage is allowing the patch to be cut to the desired size to match the size of an affected area. As such convention external patch rolls there are known types wherein a patch covered with a paper covering material is wrapped around a dispenser core, and types wherein the patch is wrapped without a covering material, such as described in Japanese Unexamined Utility Model Publication SHO No. 55-134822 and Japanese Unexamined Utility Model Publication SHO No. 56-60730.

### Disclosure of the Invention

Conventional external patch rolls, however, are associated with a problem in that they cannot maintain their initial drug effects over prolonged periods, as well as inconveniences including contamination of both sides of the roll by hand smudging during storage, and deformation of the roll shape into a thorny or bowl shape by protrusions or depressions in the wrapped sections of the patch on the perimeter of the dispenser core when the patch shifts laterally.

In light of these problems of the prior art, it is an object of the present invention to provide an external patch roll which can maintain a drug effect over prolonged periods without producing inconveniences such as roll deformation, and which permits easier attachment operations.

As a result of much diligent research aimed at achieving the aforementioned object, the present inventors have found that contributing causes of the aforementioned problems include the fact that the adhesive layer cross-section on both sides of the patch wrapped around as a roll peels off allowing the drug components to readily escape from those sections, and that the paper covering material or dispenser core tends to adsorb the drug components so that the drug components migrate and adsorb into those parts during storage. It was also found that this can be solved by using a cover formed of a material with low adsorption and permeation for drug components, for coverage of both sides of the patch wrapped around as a roll, in order to allow the drug components to be retained in the adhesive layer and the roll shape to be retained, thereby facilitating attachment operation.

In other words, the patch of the invention is characterized by being provided with a dispenser core, an external patch wrapped in a roll fashion around the perimeter side, and a pair of side covers attached to both edges of the dispenser core and covering both edge sides of the external patch, wherein the dispenser core and each of the side covers is formed of a plastic material.

Using side covers to cover both edges of the wrapped patch of the external patch roll prevents adhesion of contamination by hand smudging and the like onto both edges of the roll, inhibits escape of the drug component from both edges of the roll, and prevents deformation of both edges of the roll. In addition, providing the side covers also allows drawing out from the roll and winding up onto the roll to be accomplished in a smooth and easy manner. The dispenser core and side covers are formed of plastic materials to prevent adhesion of the drug component onto these parts.

The side covers of the external patch roll of the invention are preferably formed of polyacrylonitrile resin. The dispenser core is preferably formed of polyethylene terephthalate resin or polyacrylonitrile resin. This further prevents adsorption of the drug component onto these parts. That is because polyacrylonitrile resin and polyethylene terephthalate resin are much more resistant to adsorption and permeation of drug components than are the commonly used molding resins.

In the external patch roll of the invention, the external patch is provided with a support, an adhesive layer laminated on one side of the support, and a covering material laminated in a releasable manner on the adhesive layer, wherein the covering material is preferably a releasable film composed of polyethylene terephthalate resin. This will also allow sufficient inhibition of adsorption of the drug component onto the covering, similar to the side covers and dispenser core. The support is preferably a knitted fabric, woven fabric or nonwoven fabric composed of stretchable polyethylene terephthalate resin or polybutylene terephthalate resin. This will allow sufficient inhibition of adsorption and permeation of the drug component into the support while also maintaining adequate flexibility for the external patch.

Tearing lines are preferably formed in the external patch of the external patch roll of the invention. This will allow the patch to be easily cut to match the size of the area on which it is to be used.

More preferably, the tearing lines consist of perforation slits, with a slit width of 1.0-2.0 mm and a slit spacing of 1.0-1.5 mm, and the breaking strength when the external patch is torn off at the slits is preferably 7.36-15.24 kgf/48 mm width when a covering material is laminated on the external patch and preferably 0.76-2.65 kgf/48 mm width when no covering material is laminated on the external patch. This conveniently allows the external patch to be easily cut to match the desired size, while also preventing problems such as undesired cutting of the tearing lines during or after attachment.

Here, the "breaking strength" is the value as measured by the following method according to "Tensile breaking strength" described in JIS K7113-1981. First, a 48 mm width x 100 m length test piece is cut from the external patch. The cutting is made such that one uncut line of perforation slits is included at the center of the test piece. Next, an edge of the test piece parallel to the slits is set in a tensile tester at a grip width of 60 mm, with the center of each line of slits of the test piece and the center line of the grip of the tensile tester aligned in a straight line, the piece is pulled at a tensile speed of 500 ±50 mm/min in such a manner that the force on the test piece is applied perpendicularly to the slit line of the test piece, and the load at the point of breakage of the test piece at the slit line section is recorded as the breaking strength.

The external patch roll of the invention may also be one characterized in that the external patch contains an anti-inflammatory agent as the drug component. An external patch roll having the structural materials described above can satisfactorily employ anti-inflammatory agents with high volatility from adhesive layers and high adsorption into paper materials.

The dispenser core of the external patch roll of the invention has a cylindrical shape, and it may also have fitting members to fit each of the side covers into the dispenser core. This allows the separately manufactured side covers and the dispenser core to be easily integrated.

When the external patch roll of the invention has fitting members to allow each of the side covers to be fitted into the cylindrical dispenser core, the fitting member of each side cover preferably also has protrusions on the side. Providing protrusions on the side of the fitting member will allow the side covers and dispenser core to be more reliably and securely fitted when the side covers are fitted onto the cylindrical dispenser core.

### Brief Description of the Drawing

Fig. 1 is a general cross-sectional view of a preferred embodiment of an external patch roll according to the invention.
Fig. 2 is a general cross-sectional view of the construction of the external patch body of the external patch roll shown in Fig. 1.
Fig. 3 is a plan view of the strip-like patch body shown in Fig. 2.
Figs. 4A to 4E are magnified general cross-sectional views of external patches as seen from the direction along the tearing lines.
Fig. 5 is a perspective view of a side cover provided with protrusions on the side of the fitting member.
Fig. 6A is a front view of the side cover shown in Fig. 5, Fig. 6B is a back view of the same, Fig. 6C is a cross-sectional view along line A-A, and Fig. 6D is a cross-sectional view along line B-B of Fig. 6C.
Fig. 7 is a perspective view of an external patch roll wherein side covers such as shown in Fig. 5 are fitted into a cylindrical dispenser core.

### Best Mode for Carrying Out the Invention

A preferred embodiment of the invention will now be explained in greater detail with reference to the attached drawings. The corresponding members in the drawings are indicated by like reference numerals.

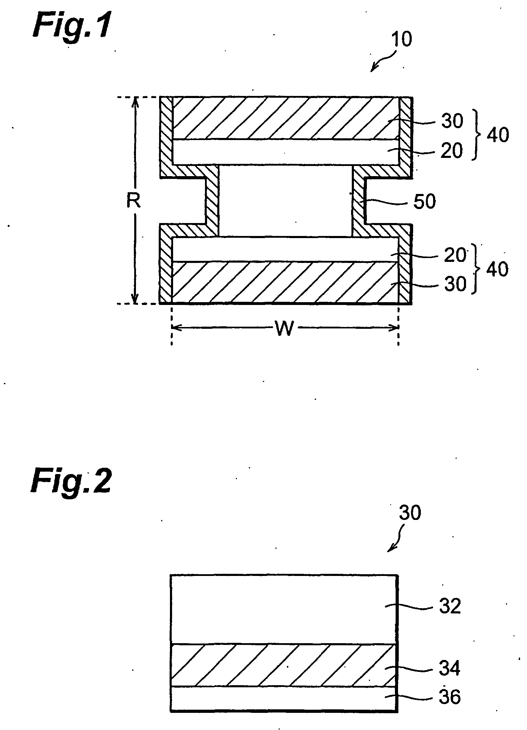

Fig. 1 is a cross-sectional view of the basic construction of a preferred embodiment of an external patch roll according to the invention, and Fig. 2 is a magnified general cross-sectional view of the basic construction of the external patch body used in Fig. 1.

As shown in Fig. 1, the external patch roll 10 of this embodiment comprises a plastic dispenser core 20, a roll 40 of an external patch 30 comprising an external patch 30 wrapped around the perimeter side of the dispenser core 20, and a pair of plastic side covers 50 attached to both edges of the dispenser core 20 and covering both edge sides of the roll 40 almost in contact therewith.

Also, as shown in Fig. 2, the external patch 30 comprises a body composed of a support 32 and an adhesive layer 34 laminated over almost the entire surface of the support 32, and a covering material 36 attached over almost the entire surface of the adhesive layer 34, to be peeled off during use.

The structural elements will now be explained in detail. The plastic dispenser core 20 has a cylindrical shape. The structural material of the dispenser core 20 is not particularly restricted so long as it is a plastic material, but it is preferably a resin with high resistance to adsorption and permeation of drug components, from which standpoint it is preferably polyethylene terephthalate (PET) resin, polyacrylonitrile (PAN) resin or the like. Selection of PET or PAN as the material is effective from the standpoint of considerably controlling loss of the drug component by the dispenser core 20, compared to selection of not only paper but also high-density polyethylene resin (HDPE) or polypropylene resin (PP), which are commonly used as molding resins.

The size of the dispenser core 20 is not particularly restricted, but the length of the dispenser core 20 is preferably equal to the width W of the roll 40. This facilitates contact of both edges of the roll 40 with the side covers 50, and is thus effective for avoiding escape of the drug component from both edges of the roll 40 and preventing shape deformation of the roll 40. The dispenser core 20 may be either colorless or colored.

The external patch 30 is in the form of a strip. One end of the strip of the external patch 30 is affixed to the outer surface of the dispenser core 30, and it is wrapped around the dispenser core 30 with the support 32 side facing outward.

The support 32 of the external patch 30 is made of a stretchable knitted fabric, woven fabric or nonwoven fabric. The structural material used for the support 32 is preferably a resin, such as PET, with very high resistance to adsorption and permeation of the drug component used in the external patch 30. This is effective for minimizing loss of the drug component by the support 32, both before use and after attachment to the affected area.

The support 32 preferably has suitable flexibility and stretchability. The thickness of the support is preferably 0.01-5 mm. If the thickness of the support 1 is less than 0.01 mm it becomes difficult to handle and tends to wrinkle, while if it exceeds 5 mm the flexibility is reduced, an uncomfortable feel is produced upon attachment, and physical irritation often results.

The structural material of the adhesive layer 34 is not particularly restricted, but it is preferably one containing the drug component and having adhesive strength allowing prolonged affixation of the drug onto the skin surface at ordinary temperature, among which there may be mentioned drug component-containing natural or synthetic rubber materials. The drug component of the adhesive layer 34 may be any drug which is percutaneously absorbed, and for example, there are preferably used anti-inflammatory agents, typically antiphlogistics or analgesics such as methyl salicylate, glycol salicylate, L-menthol, indomethacin, ketoprofen, felbinac, flurbiprofen, piroxicam, loxoprofen sodium, ibuprofen piconol, diclofenac sodium, and the like. Other drug components which may be used include antihistamines such as diphenhydramine hydrochloride, diphenhydramine salicylate, diphenylimidazole, diphenhydramine and chlorpheniramine maleate; local anesthetics such as lidocaine, tetracaine, dibucaine hydrochloride, dibucaine, ethyl aminobenzoate, procaine hydrochloride and procaine; antifungal agents such as butenafine hydrochloride, bifonazole, oxiconazole nitrate, clotrimazole, econazole nitrate, miconazole nitrate and tolnaftate; and adenocorticotropic hormone agents such as hydrocortisone acetate, betamethasone valerate, prednisolone, dexamethasone acetate, fludroxycortide, betamethasone propionate, fluocinonide and halcinonide.

The covering material 36 is a release film attached to the entire surface of the adhesive layer 34 in a releasable manner. The covering material 36 is released during use, and its function is to cover the entire surface of the adhesive layer 34 to prevent escape of the drug component and moisture in the adhesive layer 2 before the body of the external patch 30 is used, while also preventing wrinkles from occurring in the highly flexible, stretchable patch body 32. The structural material used for the covering material 36 is preferably a molding resin, such as a biaxially drawn PET film, with very high resistance to adsorption and permeation of the drug component used in the external patch 30. Selection of such a structural material can minimize loss of the drug component by the covering material 36.

The thickness of the covering material 36 is preferably 25-120 µm, and especially 50-100 µm. If the thickness of the covering material 36 is less than 25 µm it tends to be difficult to peel off from the patch, while if it is greater than 120 µm, the flexibility is reduced and it tends to become difficult to smoothly wrap around the dispenser core 20. The covering material 36 may be either colorless or colored.

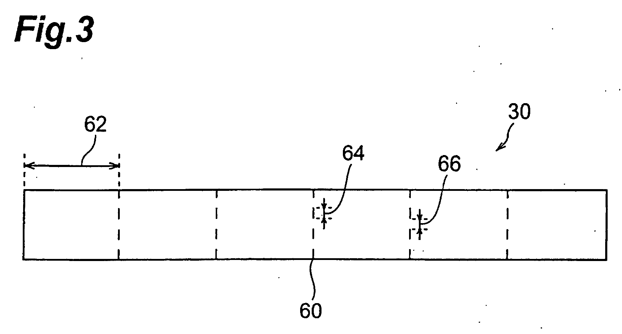

Fig. 3 is a plan view of part of the strip-like external patch 30 after it has been drawn out from the roll 40.

As shown in Fig. 3, slits 60 are formed in the external patch 30. The "slits" are formed by perforation or notch formation in the external patch 30, and Fig. 3 shows perforated slits. This allows the external patch 30 to be easily torn by hand without using a cutting tool. The attachment operation is thus facilitated.

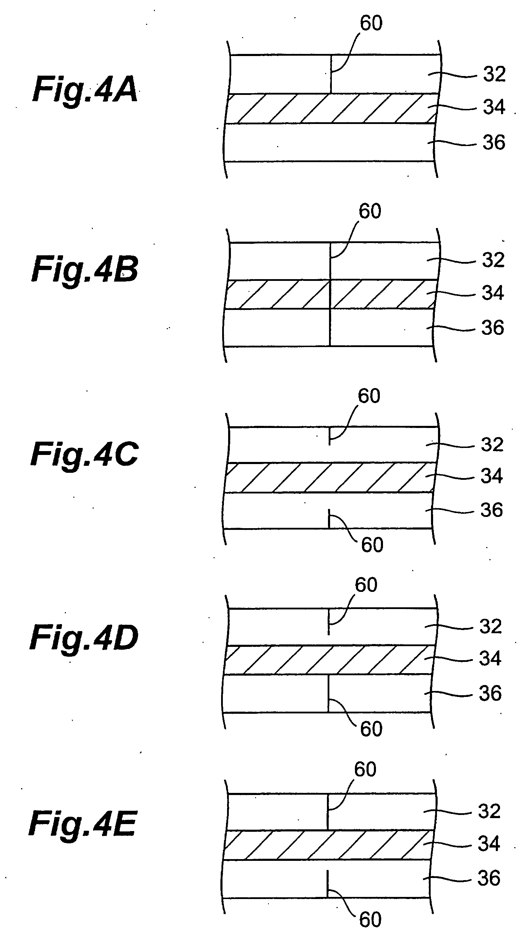

Figs. 4A to 4E are magnified general cross-sectional views of the external patch 30 as seen from the direction along the slits 60. The slits 60 may be formed only on the support 32 as shown in Fig. 4A, but they preferably run from the support 32 through the covering material 36 for ease during use, as shown in Fig. 4B. This is because when the tearing lines are formed only on the support 32, the stretchability of the patch 30 results in inconveniences during use, such as elongation of the patch 30 when cut by hand and consequent adhesion of dirt at and around the cutting holes, or remnants of only the covering material 36 after the patch body has been attached.

When the patch 30 is thus perforated to form the tearing lines, the spacing 62 between the slits 60 is not particularly restricted but is preferably no greater than 100 mm. This is in order to allow freer selection of the size of the patch to match the size of the attachment site, because when the spacing 62 between the slits 60 is greater than 100 mm, an inconvenience tends to occur in which the patch attaches not only to the desired attachment site but also onto undesired sections around it.

When the external patch 30 is used after cutting to the desired attachment area size as described above, the cut patch has a certain size and therefore will usually include several uncut perforated slits 60. From this standpoint, the slit width 64 is preferably 1.0-2.0 mm and the slit spacing (seam width) 66 is preferably 1.0-1.5 mm, while the breaking strength when the external patch is broken off at the perforation 60 is preferably 7.36-15.24 kgf/48 mm width when a covering material is laminated on the external patch and preferably 0.76-2.65 kgf/48 mm width when no covering material is laminated on the external patch.

This is because when the slit width 64 is less than 1.0 mm, the slit width 64 is relatively small compared to the seam width 66, which tends to interfere with the hand tearability of the slits 60. "Hand tearability" indicates the degree of ease with which the external patch can be easily cut at the perforated slits 60. On the other hand, if the slit width 64 is greater than 2.0 mm, the slit width 64 is, relatively large compared to the seam width 66, which is undesirable because the following two inconveniences are more likely to occur. Namely, the first inconvenience is that all or portions of the uncut slits 60 of the external patch 30 which has been cut to the desired size will be more likely to tear unexpectedly when the covering material 36 is peeled off just before use or when the used external patch 30 is peeled off from the attachment site after use. The other inconvenience is that even while attached, all or portions of the uncut slits 60 will be more likely to tear due to movement of the attachment site or even without tearing, the slit sections will be more likely to rise from the attachment site. In the following explanation, the terms "undesired cutting" and "slit rising" will be used for the former and latter cases, respectively. In addition, the slit width 64 is also preferably not larger than the seam width 66, because contaminants will tend to adhere to the cut holes of the slits 30.

The seam width 66 is also preferably not greater than the above-mentioned upper limit for the slit width 64, because the hand tearability will tend to be reduced, while it is preferably not less than the lower limit because undesired cutting and slit rising will be more conspicuous.

If the breaking strength described above is used as a measure of the degree of hand tearability, slit rising and undesired cutting, it is preferably not below the aforementioned lower limits because slit rising and undesired cutting will become more likely, and it is preferably not above the upper limits because the hand tearability is reduced.

An external patch 30 fabricated under conditions suitable for formation of the perforated slits 60 described above allows the size of the patch 30 to be selected to match the size of the attachment site and to be easily torn off, while the hand tearability is satisfactory and inconveniences such as slit rising and undesired cutting do not occur.

Some possible modifications in forming the slits 60 of Fig. 4B include forming notches while leaving a slight thickness on one or both of the supports, as shown in Figs. 4C and 4D. A "slight thickness" is a thickness which allows easy tearing by slightly bending the patch 30 or lightly pulling both ends of the notch, when the patch 30 is torn for use. Since the patch 30 is wrapped around the dispenser core 20 with the support 32 side on the outside, the modes shown in Figs. 4C and 4D can effectively prevent loss of the drug component through the cross-section of the tearing lines. These modes are especially effective in cases where very highly volatile drug components are included. When notching is employed to form the tearing lines, the modes shown in Figs. 4A and 4C to 4E are used as the tearing lines. By setting the "slight thickness" to a suitable size, it is possible to achieve the same satisfactory hand tearability as with the aforementioned perforated slits, and thus prevent inconveniences such as slit rising and undesired cutting.

The plastic side covers 50 are panels provided with protrusions (fitting members) in the center, as shown in Fig. 1, and the protrusions (fitting members) are preferably fitted onto both ends of the dispenser core 20 in such a manner as to contact the inner perimeter of the cylindrical dispenser core 20. Each of the side covers 50 is fitted onto the dispenser core 20 while the surface surrounding the protrusion (fitting member) is almost in contact with the edge of the roll 40. The edges of the roll 40 are thus covered by the side covers, so that escape of the drug component from both edges of the roll 40 can be inhibited while also preventing shape deformation of the roll 40 as well as adhesion of contaminants by hand smudging and the like. Providing side covers during use as described below also facilitates drawing out and winding up of the external patch 30.

The structural material of the side covers 50 is not particularly restricted so long as it is a plastic material, but it is preferably a resin with high resistance to adsorption and permeation of drug components, from which standpoint PAN is preferred. Selection of PAN as the material is effective from the standpoint of considerably controlling loss of the drug component through the side covers 50, compared to selection of not only paper but also HDPE, PP or the like, which are commonly used as molding resins.

The outer diameter R of each side cover 50 is preferably of a greater size than the outer diameter r (not shown) of the roll 40. This is effective from the standpoint of allowing the total surface of each edge of the roll 40 to be covered and thus inhibiting escape of the drug component. Each side cover 50 also preferably has an appropriate thickness for the structural material used, so that it does not easily deform, in order to effectively prevent shape deformation of the roll 40.

The method of using the rolled patch 10 described above is fundamentally the same as for conventional rolled patches. First, the rolled patch 10 is held with one hand, and the patch 30 is drawn out from the roll 40 by gripping with the fingers of the other hand. Here, the side covers 50 serve as guides to hold both edges of the patch 30 strip being drawn out from the roll 40, to allow the patch 30 to be drawn out in a smooth and straight manner from the roll 40. Also, since the fingers of the hand holding the rolled patch 10 do not touch the edges of the roll 40 with the exposed adhesive layer 34, as a result of the side covers, there is no such inconvenience as contamination of these sections by hand smudging, as occurs with the prior art. In addition, the covering material 36 attached to the patch 30 provides suitable stiffness to the patch 30, thus eliminating inconveniences such as stretching and wrinkling of the support 32 even when relatively strong pulling force is used for drawing out.

The drawn out patch 30 strip is cut to a size matching the affected area. Because of the notching formed in the support 32 and covering material 34 of the patch 30 in order to maintain satisfactory hand tearability and prevent the inconveniences of slit rising and undesired cutting, the tearing can be easily accomplished without a cutting tool such as a cutter or scissors for adjustment to the desired size. The lack of need for a cutting tool also improves the safety.

The cut patch 30 is then bent slightly so that the edge section of the film of the covering material 36 laminated in a releasable manner on the adhesive layer 34 is raised from the adhesive layer 34. The raised edge section of the covering material 36 is gripped with the fingers and part of the covering material 36 is peeled from the adhesive layer 34.

Next, the body of the patch 30 from which the covering material 36 has been peeled is attached to the affected area. When part of the covering material 36 has been peeled from the adhesive layer 34, the exposed section of the adhesive layer 34 of the patch is used for temporary attachment onto the prescribed affected area, and then the temporary attachment section is pressed with the finger while the rest of the covering material 36 is slowly peeled to avoid wrinkling and the patch 30 is attached. Alternatively, the entire covering layer 36 may be peeled off from the adhesive layer 34 first, and then the body of the patch 30 attached to the affected area.

Finally, the unused section of the patch 30 which has been drawn out from the roll 40 is wrapped back onto the roll 40. Here, as for drawing out, the side covers 50 again serve as guides to hold both edges of the patch 30 strip which have been drawn out, thus allowing the patch 30 to be wrapped up neatly and easily. Shape deformation of the roll 40 due to repeated use can therefore be prevented. Since the attached patch is notched to prevent the inconveniences of slit rising and undesired cutting, such inconveniences do not occur either during attachment or upon peeling after use.

During storage, the rolled patch 10 has both edges of the roll 40 covered by the pair of side covers 50 almost in contact therewith, and therefore escape of the drug component to the outside from the side edges of the roll 40 is prevented, while shape deformation of the roll 40 is also prevented. In addition, since all of the structural members of the rolled patch 10 except for the adhesive layer 34 are formed from a material. with very high resistance to adsorption and permeation of the drug component, including the side covers 50, loss of the drug component during storage due to adsorption and permeation into the'structural members is minimized. This allows the rolled patch 10 to maintain the original drug component content with virtually no reduction. For example, even when the rolled patch 10 is carried while walking, etc. and placed in an environment with a relatively high temperature, there is virtually no shape deformation, contamination or reduction in drug effect of the roll 40 as compared with conventional types, and it may be used by easily tearing to match the size of the site of use when necessary.

Preferred embodiments of the invention have been explained above, but the invention is not limited to these embodiments. For example, although the above explanation was concerned with a plastic dispenser core with a cylindrical shape, it may be rod-shaped instead of cylindrical. The dispenser core and side covers may also be integrally formed. So long as the outer surface of the dispenser core and side covers which is exposed to contact with the drug component is covered with plastic, the interior may even be made of metal, wood, paper, ceramic or the like.

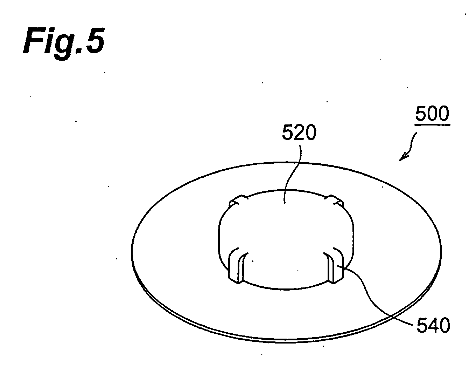

The embodiments described above also concerned external patches employing side covers with fitting members, but the shape of the side covers used for the external patch of the invention is not limited thereto. For example, the side covers with fitting members may also have protrusions on the sides of the fitting members, as will be explained below with reference to Figs. 5, Figs. 6A to 6D and Fig. 7.

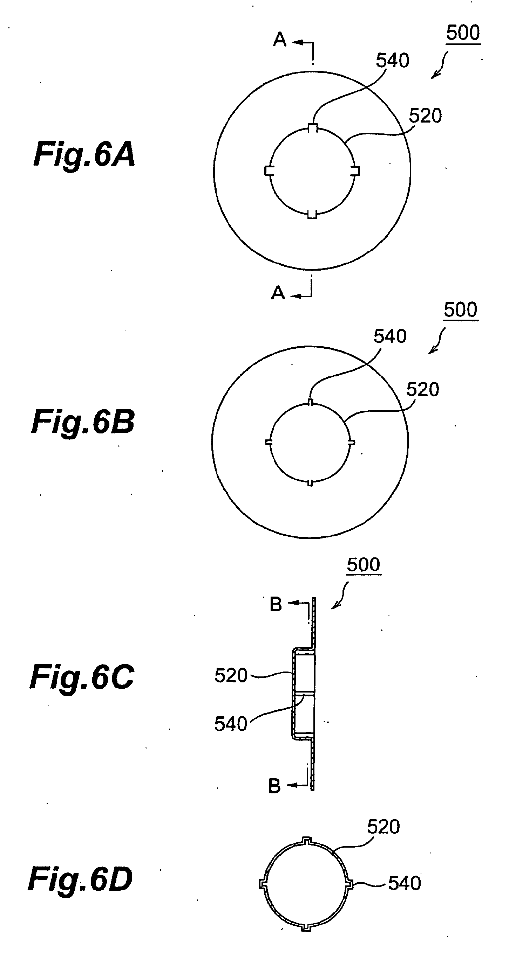

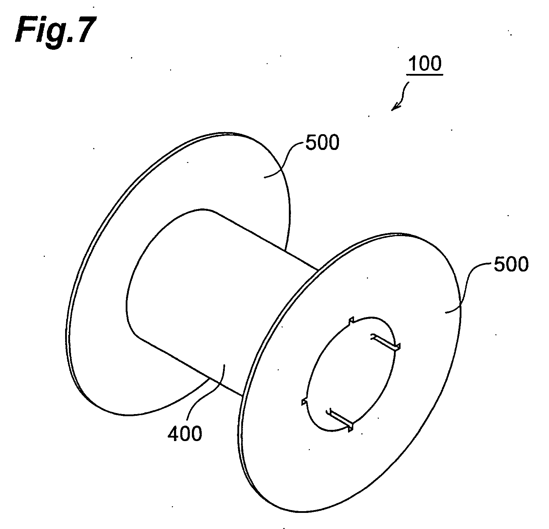

Fig. 5 is a perspective view of a side cover 500 provided with four protrusions 540 on the side of the fitting member 520. Fig. 6A is a front view of the side cover 500 shown in Fig. 5, Fig. 6B is a back view of the side cover 500 shown in Fig. 5, Fig. 6C is a cross-sectional view along line A-A of the side cover 500 shown in Fig. 5, and Fig. 6D is a cross-sectional view along line B-B of Fig. 6C. Fig. 7 is a perspective view of an external patch roll 100 wherein side covers 500 such as shown in Fig. 5 are fitted into a cylindrical dispenser core 400. By thus providing protrusions 540 on the sides of the fitting members 520, the protrusions 540 contacting the inner surface of the dispenser core 400 press onto the inner surface of the dispenser core 400 from the inside when the side covers are fitted onto the cylindrical dispenser core 400 as shown in Fig. 7, thus resulting in more reliable and secure fitting of the side covers 500 with the dispenser core 400. The number of protrusions is not particularly restricted. For example, a single protrusion may be sufficient so long as it allows reliable and secure fitting of the side covers and the dispenser core.

The rolled patch of the invention will now be explained in greater detail by way of examples and comparative examples, with the understanding that the invention is in no way limited by these examples.

### [Example 1]

A PET cylinder (outer diameter: 27.7 mm, height: 48.0 mm) was used as a dispenser core. As the external patch there was used an antiphlogistic analgesic patch comprising a three-layer structure with a support, adhesive layer and covering material, having a width of 48.0 mm, a length of 3.0 m and a total three-layer thickness of 0.72 mm. The support of the antiphlogistic analgesic patch was a PET thin film (550 µm thickness), the adhesive layer was composed mainly of styrene-isoprene-styrene block copolymer, the drug component was a thin layer containing glycol salicylate (content: 5.0 g/m²), and the covering material was a PET thin film (75 µm thickness). The side covers were made of PAN (ZEXLON, product of Mitsui Chemical Corp.) each in the shape of a disk (cover outer diameter: 60 mm; thickness: 0.3 mm), with protrusions in the center allowing them to be fitted into the cylinder of the dispenser core. The side covers were attached onto the dispenser core and the stretchable patch was wound around the outer surface of the dispenser core to prepare a rolled antiphlogistic analgesic external patch.

### [Example 2]

A rolled antiphlogistic analgesic external patch was prepared in the same manner as Example 1, except that the dispenser core was made of PAN.

### [Example 3]

A rolled antiphlogistic analgesic external patch was prepared in the same manner as Example 1, except that the side covers were made of PET and the dispenser core was made of HDPE.

### [Example 4]

A rolled antiphlogistic analgesic external patch was prepared in the same manner as Example 1, except that the side covers were made of PET and the dispenser core was made of PP.

### [Example 5]

A rolled antiphlogistic analgesic external patch was prepared in the same manner as Example 1, except that the side covers were made of PET.

### [Example 6]

A rolled antiphlogistic analgesic external patch was prepared in the same manner as Example 1, except that the side covers were made of PET and the dispenser core was made of PAN.

### [Example 7]

A rolled antiphlogistic analgesic external patch was prepared in the same manner as Example 1, except that the dispenser core was made of HDPE.

### [Example 8]

A rolled antiphlogistic analgesic external patch was prepared in the same manner as Example 1, except that the dispenser core was made of PP.

### [Example 9]

A rolled antiphlogistic analgesic external patch was prepared in the same manner as Example 1, except that indomethacin (content: 1.0 g/m²) was used as the drug component.

### [Example 10]

A rolled antiphlogistic analgesic external patch was prepared in the same manner as Example 1, except that ketoprofen (content: 1.0 g/m²) was used as the drug component.

### [Example 11]

A rolled antiphlogistic analgesic external patch was prepared in the same manner as Example 1, except that felbinac (content: 1.0 g/m²) was used as the drug component.

### [Example 12]

A rolled antiphlogistic analgesic external patch was prepared in the same manner as Example 1, except that flurbiprofen (content: 1.0 g/m²) was used as the drug component.

### [Example 13]

A rolled antiphlogistic analgesic external patch was prepared in the same manner as Example 1, except that loxoprofen sodium (content: 1.0 g/m²) was used as the drug component.

### [Example 14]

A rolled antiphlogistic analgesic external patch was prepared in the same manner as Example 1, except that diclofenac sodium (content: 1.0 g/m²) was used as the drug component.

### [Comparative Example 1]

A rolled antiphlogistic analgesic external patch was prepared in the same manner as Example 1, except that the side covers were made of PET and the dispenser core was made of paper.

### [Comparative Example 2]

A rolled antiphlogistic analgesic external patch was prepared in the same manner as Example 1, except that no side covers were attached, and the dispenser core was made of paper.

### [Comparative Example 3]

A rolled antiphlogistic analgesic external patch was prepared in the same manner as Example 1, except that no side covers were attached, and the dispenser core was made of HDPE.

### [Comparative Example 4]

A rolled antiphlogistic analgesic external patch was prepared in the same manner as Example 1, except that no side covers were attached, and the dispenser core was made of PP.

### [Comparative Example 5]

A rolled antiphlogistic analgesic external patch was prepared in the same manner as Example 1, except that no side covers were attached.

### [Comparative Example 6]

A rolled antiphlogistic analgesic external patch was prepared in the same manner as Example 1, except that no side covers were attached, and the dispenser core was made of PAN.

The rolled patches of Examples 1-8 and Comparative Examples 1-6 were subjected to the following two tests.

### <Drug effect retention test>

The rolled patches of Examples 1-8 and Comparative Examples 1-6 were packed into aluminum drug packing paper and stored in an environment at a temperature of 40°C and a relative humidity of 75%, for an acceleration test to determine the amount of reduction in drug component in the adhesive layer, and the amount of glycol salicylate remaining as the drug component was measured after 6 months. The results are shown in Table 1 below in terms of the proportion of remaining drug with respect to the initial content. If the proportion of remaining drug with respect to initial content of the drug component is at least 95 wt% after 6 months under the conditions for this acceleration test, then a drug effect of 3 years can be guaranteed under conditions of normal use, i.e. at room temperature.

The test results for the samples are shown in Table 1, based on the following evaluation standard: 4 = 95 wt% or greater, 3 = at least 90 wt% and less than 95 wt%, 2 = at least 85 wt% and less than 90 wt%, 1 = less than 85 wt%.

### <Convenience during use>

The samples of Examples 1-8 and Comparative Examples 1-6 were used by 20 persons complaining of shoulder stiffness or muscle soreness, at their own discretion for a period of one week, and an evaluation was made in regard to inconveniences such as lateral sliding of the patch during storage, roll deformation during use, including formation of thorny or bowl shapes produced by repeated drawing out and winding up of the patch during use, and contamination of the edges of the external patch wrapped up into a roll.

The test results for the samples are shown in Table 1, based on the following evaluation standard: 2 = no inconvenience; 1 = inconvenience

**Table 1**

| | Side covers | Dispenser core | Drug effect retention | Convenience during use |
|---|---|---|---|---|
| Example 1 | PAN | PET | 4 | 2 |
| Example 2 | PAN | PAN | 4 | 2 |
| Example 3 | PET | HDPE | 3 | 2 |
| Example 4 | PET | PP | 3 | 2 |
| Example 5 | PET | PET | 3 | 2 |
| Example 6 | PET | PAN | 3 | 2 |
| Example 7 | PAN | HDPE | 3 | 2 |
| Example 8 | PAN | PP | 3 | 2 |
| Comp. Ex. 1 | PET | Paper | 1 | 2 |
| Comp. Ex. 2 | none | Paper | 2 | 1 |
| Comp. Ex. 3 | none | HDPE | 2 | 1 |
| Comp. Ex. 4 | none | PP | 2 | 1 |
| Comp. Ex. 5 | none | PET | 3 | 1 |
| Comp. Ex. 6 | none | PAN | 3 | 1 |

As is clear from the results shown in Table 1, the rolled antiphlogistic analgesic external patches of the invention provided with plastic side covers and a plastic dispenser core in Examples 1-8 allowed control of drug component loss while also preventing shape deformation of the rolls and adhesion of contaminants due to hand smudging, and permitting smooth drawing out and winding up operations of the patches from and onto the rolls.

In particular, the rolled antiphlogistic analgesic external patches of Examples 1 and 2, corresponding to the preferred embodiments of the invention with the side covers made of PAN and the dispenser core made of PET or PAN, exhibited values of 95% or greater as the proportion of drug component remaining after 6 months with respect to the initial drug component amount under environmental conditions of 40°C temperature and 75% relative humidity. This demonstrated that the drug effects of these external patches are guaranteed to be effectively maintained for 3 years at room temperature. The external patches of Examples 1 and 2 were also confirmed to produce no inconveniences such as contamination of both edges of the external patches when wound up as a roll or roll shape deformation either during use or during storage, in comparison to the prior art samples.

### <Test of convenience during use with perforation tearing lines>

The external patches prepared in Example 1 above were subjected to perforation working to prepare samples 1 to 12 having the slit widths and seam widths shown in Table 2.

Samples 1 to 12 were cut into 48 mm width x 100 m length test pieces, and the breaking strength at the perforations (tearing lines) was measured with a tensile tester under the conditions described above. The breaking strength was measured for each test piece both when covered with a covering material and with the covering material peeled off.

Samples 1 to 12 were also used by 20 persons for 7 days for evaluation of the convenience during use ("hand tearability", "undesired cutting" and "slit rising").

The values for the breaking strengths of samples 1 to 12 and the results of evaluating the usability are shown in Table 2. The evaluation of the usability was as follows.

### "Hand tearability"

2 = Patch easily tearable by hand at perforation, slit cross-section clean, no problems for practical use
1 = Patch not easily tearable by hand at perforation, slit cross-section rough, problems for practical use

### "Undesired cutting"

2 = No undesired cutting at uncut slits when covering material peeled just before use or when used patch peeled from skin
1 = Undesired cutting at uncut slits when covering material peeled just before use or when used patch peeled from skin

### "Slit rising"

2 = No undesired cutting at uncut slits due to movement of attachment site during use
1 = Undesired cutting at uncut slits due to movement of attachment site during use

**Table 2**

| Perforations | | | Breaking strength (kgf/48 mm width) | | Convenience during use | | |
|---|---|---|---|---|---|---|---|
| Sample | Slit width (mm) | Seam width (mm) | With covering material | Without covering material | Hand tearability | Undesired cutting | Slit rising |
| 1 | 4.0 | 1.0 | 3.80 | 0.50 | 2 | 1 | 1 |
| 2 | 3.0 | 1.0 | 5.26 | 0.63 | 2 | 1 | 1 |
| 3 | 2.0 | 1.0 | 7.36 | 0.76 | 2 | 2 | 2 |
| 4 | 1.8 | 1.0 | 11.00 | 0.91 | 2 | 2 | 2 |
| 5 | 1.5 | 1.0 | 12.34 | 1.08 | 2 | 2 | 2 |
| 6 | 1.0 | 1.0 | 15.24 | 1.28 | 2 | 2 | 2 |
| 7 | 0.7 | 1.0 | 17.00 | 1.40 | 1 | 2 | 2 |
| 8 | 2.0 | 0.7 | 3.43 | 0.47 | 2 | 1 | 1 |
| 9 | 2.0 | 1.0 | 7.36 | 0.76 | 2 | 2 | 2 |
| 10 | 2.0 | 1.2 | 10.05 | 1.50 | 2 | 2 | 2 |
| 11 | 2.0 | 1.5 | 12.90 | 2.65 | 2 | 2 | 2 |
| 12 | 2.0 | 1.7 | 16.20 | 3.69 | 1 | 2 | 2 |

As is clear from the results shown in Table 2, it was confirmed that when perforation slits were formed in the patch, and the slit width is 1.0-2.0 mm, the slit spacing is 1.0-1.5 mm, the breaking strength is 7.36-15.24 kgf/48 mm width for tearing of the external patch at the perforation when a covering material is laminated on the external patch and the breaking strength is 0.76-2.65 kgf/48 mm width when no covering material is laminated on the external patch, the external patch can be easily torn to match the desired size with no inconvenience, while the hand tearability is satisfactory, and problems such as undesired cutting of the tearing lines during or after attachment are prevented.

### Industrial Applicability

As explained above, the rolled patch of the invention is able to avoid lateral sliding of the external patch wound up in a roll fashion, to prevent adhesion of contaminants onto both edge sides of the external patch wound up in a roll fashion, to control loss of drug components during storage and to achieve smooth drawing out and winding up of the patch, and therefore provides an external patch roll which eliminates problems such as roll deformation and the like, maintains drug effects for prolonged periods and allows attachment operations to be carried out in an easy manner.

## Claims

1. An external patch roll provided with
a dispenser core,
an external patch wrapped in a roll fashion around the perimeter side of said dispenser core, and
a pair of side covers attached to both edges of said dispenser core and covering both edge sides of said external patch,
wherein said dispenser core and each of said side covers is formed of a plastic material.

2. An external patch roll according to claim 1,
wherein each of said side covers is formed from polyacrylonitrile resin, and said dispenser core is formed from polyethylene terephthalate resin or polyacrylonitrile resin.

3. An external patch roll according to claim 1,
wherein said external patch is provided with a support, an adhesive layer laminated on one side of said support and a covering material laminated in a releasable manner on said adhesive layer, and said covering material is a release film made of polyethylene terephthalate resin.

4. An external patch roll according to claim 1,
wherein said external patch is provided with a support and an adhesive layer laminated on one side of said support, and said support is a knitted fabric, woven fabric or nonwoven fabric composed of stretchable polyethylene terephthalate resin or polybutylene terephthalate resin.

5. An external patch roll according to claim 1,
wherein tearing lines are formed in said external patch.

6. An external patch roll according to claim 1,
wherein said tearing lines consist of perforation slits, with a slit width of 1.0-2.0 mm and a slit spacing of 1.0-1.5 mm, and the breaking strength when said external patch is torn off at said slits is 7.36-15.24 kgf/48 mm width when a covering material is laminated on said external patch and 0.76-2.65 kgf/48 mm width when no covering material is laminated on said external patch.

7. An external patch roll according to claim 1,
wherein said external patch contains a drug which is percutaneously absorbed as the drug component.

8. An external patch roll according to claim 7,
wherein said drug which is percutaneously absorbed is an anti-inflammatory agent.

9. An external patch roll according to claim 1,
wherein said dispenser core has a cylindrical shape, and each of said side covers has a fitting member for fitting into said dispenser core having a cylindrical shape.

10. An external patch roll according to claim 9,
wherein the fitting member of each of said side covers has protrusions on its side.
